Europäisches Patentamt

European Patent Office

Office européen des brevets

(11) Veröffentlichungsnummer: **0 109 931**

A2

(12) **EUROPÄISCHE PATENTANMELDUNG**

(21) Anmeldenummer: **83810491.7**

(22) Anmeldetag: **24.10.83**

(51) Int. Cl.³: **C 07 C 85/24**
**C 07 C 87/50**

(30) Priorität: **25.10.82 CH 6188/82**
**14.03.83 CH 1368/83**

(43) Veröffentlichungstag der Anmeldung:
**30.05.84 Patentblatt 84/22**

(84) Benannte Vertragsstaaten:
**BE DE FR GB IT**

(71) Anmelder: **ELPROCHINE AG**
**Hochstrasse 28**
**CH-8044 Zürich(CH)**

(72) Erfinder: **Biller, Efim**
**Hochstrasse 28**
**CH-8044 Zürich(CH)**

(54) Verfahren zur Herstellung von Methylenbrücken aufweisenden Polyarylaminen.

(57) Die Herstellung von Gemischen aus Methylendianilin und oligomeren Polymethylen-Polyphenylen-Polyaminen aus Anilin und Formaldehyd in Anwesenheit von Säurekatalysatoren mit steuerbarem Gehalt an Isomeren in Zweikern und an Methylendianilin in Gesamtgemisch und/oder reduzierter Säurekatalysatormenge erfolgt durch Aufteilung der Umsetzung in mindestens zwei Ströme: der eine katalytisch, der andere nicht-katalytisch, die anschliessend gegebenenfalls nach Zwischenumlagerung und gegebenenfalls nach weiterer Formaldehyd-Zugabe gemeinsam in einer oder mehreren Stufen zu gewünschten Produkten umgelagert werden.

EP 0 109 931 A2

ELPROCHINE AG, Zürich, Schweiz

VERFAHREN ZUR HERSTELLUNG VON METHYLENBRÜCKEN AUFWEISENDEN POLYARYLAMINEN

Die Herstellung von Polyaminen der Diamino-diarylmethan-Reihe durch Kondensation von aromatischen Aminen mit Formaldehyd in Gegenwart von sauren Katalysatoren ist bekannt und liefert je nach Verfahrensweise ein unterschiedlich zusammengesetztes Produkt. So erhält man bei der Kondensation in Gegenwart von schwach sauren oder Spuren von stark sauren Katalysatoren Polyamingemisch mit hohem Anteil an 2,4'-Diamino-diarylmethanen, während Polyamine mit hohem Anteil an 4,4'-Diamino-diarylmethanen und gleichzeitig geringem Gehalt an 2,4'-Isomeren nur in Gegenwart von grösseren Mengen stark saurer Katalysatoren hergestellt werden können. Für den letzten Fall am besten geeignet sind starke Mineralsäuren, insbesondere Salzsäure (DOS 1 518 406; DOS 2 045 834; US P 3 367 969).

Der Vorteil der hohen Selektivität der stark sauren Mineralsäuren für die Bildung von 4,4'-Isomeren muss jedoch nach den bekannten Methoden des Standes der Technik mit dem Verlust des Katalysators erkauft werden, da dieser nach Beendigung der Reaktion durch aufwendige Neutralisation mit Basen aus dem Reaktionsgemisch entfernt werden muss. Da eine sinnvolle Verwertung der Neutralisationssalze nicht möglich ist, bereiten diese beträchtliche Umweltprobleme.

Die Methylenbrücken aufweisenden Polyamine werden bei der Herstellung von Isocyanaten gebraucht. Die Isocyanate wiederum werden zur Herstellung von verschiedenen Polyurethanen, Polyisocyanaten und anderen zelligen und nichtzelligen Polymerisaten, die von Polyisocyanaten ableitbar sind, gebraucht.

0109931

Die Eigenschaften obiger Isocyanate sind von der Zusammensetzung der Isomeren in monomerem Methylendianilin (MDA) sowie oligomeren Polymethylen-Polyphenylen-Polyaminen (PMPPA) in anilinfreiem Polyamin-Gemisch abhängig.

Da die Marktanforderungen nicht gleichbleibend sind, ist es erwünscht, in einer Anlage Polyamin-Gemische herzustellen, die dem Bedarf angepasst werden können.

Gegenstand der vorliegenden Erfindung ist somit ein Verfahren zur Herstellung von Gemischen aus MDA und PMPPA mit steuerbarem Gehalt an 2,4'-MDA in Zweikern und steuerbarem Gehalt an MDA im Gesamt-Gemisch und/oder reduzierter Säurekatalysatormenge, das dadurch gekennzeichnet ist, dass man die Umsetzung der Ausgangsstoffe auf verschiedene Weise durchführt, indem man

a) einen Teil Formaldehyd und Anilin in Anwesenheit von gegebenenfalls gesamtem Säurekatalysator zu saurem Kondensat, im wesentlichen bestehend aus Aminobenzylanilin und/oder Methylendianilin und oligomerem Polymethylen-Polyphenylen-Polyaminen umsetzt;

b) das restliche Anilin und Teil oder das gesamte restliche Formaldehyd entweder nicht katalytisch zu sogenanntem Vorkondensat umsetzt, gegebenenfalls das Formaldehyd und Reaktionswasser abtrennt und gegebenenfalls mit dem Rest des Säurekatalysators, einen Teil oder das gesamte saure Kondensat zum Zwischenkondensat umlagert oder katalytisch direkt zum Zwischenkondensat in Anwesenheit von schwachen Säuren, Kohlensäure, sauren Ionenaustauschern, festen Katalysatoren, Salzen, starken Säuren mit kurzen Verweilzeiten, thermisch bei Temperaturen über 100°C umsetzt und

c) das saure Kondensat und Vorkondensat oder das Zwischenkondensat gegebenenfalls zusammenmischt und gegebenenfalls nach Zugabe von Restformaldehyd, gegebenenfalls
mehrstufig zu Methylendianilin sowie oligomerem Polymethylen-Polyphenylen-Polyaminen umlagert und nach dem
Stand der Technik aufarbeitet.

Die Umsetzung von Anilin und Formaldehyd in Anwesenheit von
starken Säuren wurde bereits ausführlich untersucht. Diese
erfolgt in drei Stufen, wobei in Anwesenheit von starken
Säuren die in der ersten Stufe entstandenen Verbindungen
der Methylendianilin- bzw. Anhydroformaldehydanilinart
(auch Vorkondensate genannt) sich sofort in sogenannte N-
Phenylaminobenzylamine (ABA) umlagern.

Am Ende der Reaktion lagern sich obige Aminobenzylaniline
in die entsprechenden MDA- und PMPPA-Verbindungen, wobei für
die weitere Umsetzung mit Phosgen wichtig ist, dass diese
vollständig ist: die Alkylengruppen müssen praktisch ausschliesslich mit den Kohlenstoffatomen der aromatischen
Ringe verknüpft sein.

Technisch wird das gesamte Formaldehyd mit Anilin und Säurekatalysatoren in mehreren Temperaturstufen umgesetzt, wobei
die erste im allgemeinen bei unter etwa 60$^{o}$C erfolgt. Wegen
grosser Exothermie gibt es verschiedene Verfahren zum Vermeiden von lokalen Ueberhitzungen (DP 16 43 363).

Es sind auch Verfahren bekannt, die durch Zugabe eines Teils
Anilin in das mehr oder weniger gebildete ABA/MDA/PMPPA die
Bildung von MDA begünstigen sollen (US P 3 367 969).

Weiter sind Verfahren bekannt, bei denen das gesamte Anilin
und Formaldehyd ohne Säurekatalysatoren (nichtkatalytisch) zu
sogenannten Vorkondensaten umsetzt und gegebenenfalls nach

Abtrennung von Formaldehyd und Reaktionswasser mit Säuren, Säuresalzen und ähnlichen oder festan Katalysatoren (Kationenaustauscher, Tonerden u.ä.m.) über ABA direkt oder indirekt zu MDA/PMPPA-Gemischen umlagert. Die so erhaltenen Produkte unterscheiden sich von Produkten der Umsetzung in Anwesenheit von starken Säuren bezüglich der Isomerenverteilung des MDA und des Verhältnisses MDA zu PMPPA beträchtlich; ausserdem bereitet es Schwierigkeiten, nach diesem Verfahren Produkte zu erhalten, die von polymeren, sekundären Aminen frei sind (NMR-Analyse).

Es wurde nun gefunden, dass es möglich ist, die Zusammensetzung der Reaktionsprodukte der Kondensation von Anilin mit Formaldehyd zu steuern, wenn man die Umsetzung in mindestens zwei Ströme aufteilt:

Einen Teil Anilin und Formaldehyd setzt man mit gegebenenfalls gesamter Menge Säurekatalysator in einer oder mehreren Stufen nach dem Stand der Technik zum sauren Kondensat um, das im wesentlichen aus ABA und/oder MDA/PMPPA besteht.

Unter Säurekatalysator versteht man wasserlösliche Säuren mit einem unter 2,5 liegenden pKA-Wert wie z.B. Salzsäure, Schwefelsäure, Methansulfonsäure, Trifluoressigsäure, Bromwasserstoffe, Hydroxyäthansulfonsäure u.ä.

Beim Arbeiten mit Neutralisation des Katalysators (z.B. mit Salzsäzre) wird diese in wässerige Lösung direkt oder als wässerige Lösung von Anilinhydrochlorid angewendet.

Beim Aufarbeiten der Reaktionsgemische durch Austausch mit Anilin (DOS 26 48 982) oder Extraktion mit hydrophoben Lösungsmitteln (DAS 22 38 920) erhält man den Säurekatalysator als wässerige Aminsalzlösung.

Das restliche Anilin und Formaldehyd kann nichtkatalytisch bei Temperaturen von 5-100$^{\circ}$C, vorzugsweise 20-80$^{\circ}$C, zum Vorkondensat umgesetzt werden. Dieses ist nach Abtrennung von Reaktionswasser und/oder Formaldehydwasser in Abhängigkeit vom Molverhältnis Anilin/Formaldehyd eine mehr oder weniger viskose Flüssigkeit, die bei Temperaturen von unter 40$^{\circ}$C zu Kristallbildung neigt. Das Vorkondensat kann als Emulsion (ohne Wasserabtrennung) oder nach einfacher Dekantierung des Wassers verarbeitet werden.

Dieses Vorkondensat wird auf verschiedene Weise zu Zwischenkondensat umgelagert:

Beim Arbeiten mit konzentrierten Säurekatalysatoren wie Salzsäure kann man das Vorkondensat bei Temperaturen von unter 60$^{\circ}$C mit dem Rest des Säurekatalysators, einem Teil oder dem gesamten sauren Kondensat in praktisch beliebigem Verhältnis mischen, wobei keine oder nur fein dispergierte Feststoffe sich bilden. Dies ist von Bedeutung für die Produktqualität.

Das Arbeiten bei niedriger Temperatur liefert Produkte mit minimalem (unter 1%) N-Methylgehalt.

Beim Arbeiten mit verdünnten Salzlösungen erhält man homogene Mischungen erst bei höheren Temperaturen (über 60$^{\circ}$C). Unter diesen Bedingungen bilden sich aber höhere Mengen an N-Methylverbindungen. Dies kann vermieden werden, wenn man das Vorkondensat bei Temperaturen von unter 60$^{\circ}$C mit relativ kleinen Mengen an Restkatalysator oder saurem Kondensat zu im wesentlichen ABA umlagert und erst dann bei Temperaturen von über 60$^{\circ}$C mit dem Rest des sauren Kondensats zusammenmischt: man erhält homogene Lösungen und nach Endumlagerung Produkte, ohne dass übermässige Mengen an N-Methylverbindungen sich bilden.

Das restliche Anilin und Formaldehyd können auch direkt zu Zwischenkondensat umgesetzt werden. Dies kann auf verschiedene Weise erfolgen, z.B. kann Anilin und Formaldehyd in Anwesenheit von schwachen Säuren, sauren Ionenaustauschern, basischen Salzen oder mit starken Säuren und kurzen Verweilzeiten, thermisch bei Temperaturen von über 100°C oder heterogen mit wässerigen Säuren umgesetzt werden.

Das Isomerenverhältnis in Zweikern (MDA) sowie das Verhältnis von MDA zu PMPPA kann auf verschiedene Weise geregelt bzw. eingestellt werden:

a) durch mengemässige Einstellung der Ströme;
b) durch verschiedene Anilin/Formaldehyd Molverhältnisse in den Strömen;
c) durch die Säurekatalysatormenge im sauren Kondensat (Protonierungsgrad);
d) durch die Katalysatormenge und Temperaturbedingungen bei der Herstellung von Zwischenkondensat;
e) durch Arbeiten mit mehr als zwei Strömen, und
f) durch Bedingungen, unter denen die Ströme vor der Endumlagerung behandelt werden bzw. unter denen die Mischung verschiedener Ströme erfolgt.

Ueberraschenderweise wurde gefunden, dass das durch nichtkatalytische Umsetzung von Anilin und Formaldehyd hergestellte Vorkondensat bei Mischen mit saurem Kondensat sich ganz anders verhält als bei der Formaldehydzugabe nach dem Stand der Technik zu Anilinsalzlösungen, die ähnliche Protonierungsgrade aufweisen.

Nach dem erfindergemässen Verfahren erhält man beim Mischen von saurem Kondensat mit Vorkondensat und einem Endprotonierungsgrad von unter 0,2 Mol Säure/pro Mol Anilin Lösungen ohne nennenswerte Feststoffbildung bei Temperaturen von unter 55°C.

Von Bedeutung ist die Konzentration des Wassers in dem sauren Kondensat. Je höher die Wassermenge ist, umso höher ist die Temperatur, bei der das Zwischenkondensat sich homogen, praktisch feststofffrei, mit saurem Kondensat löst.

Die Tatsache, dass Polyamine aus Umsetzung in Anwesenheit von grossen Mengen an starken Säuren Produkte mit über 95% 4,4'-MDA in Zweikern und die Polyamine aus der Umlagerung der Vorkondensate in Abhängigkeit von Säurekatalysatoren nach ihrer Art und ihrer Menge (z.B. DOS 23 01 554) Polyamine mit einem hohen Gehalt an 2,4'-MDA liefern, erlaubt durch das erfindungsgemässe Verfahren die Produktzusammensetzung zu steuern und die dazu notwendige Katalysatormenge im Vergleich zum Stand der Technik erheblich zu reduzieren.

Bei Herstellung von Methylenbrücken aufweisende Polyarylamine, die beträchtliche Mengen an Tri-, Tetra- und höheren Polyarylaminen (PMPPA) enthalten, können erhalten werden, wenn man insbesondere niedere Anilin/Formaldehyd-Molverhältnisse, wie z.B. bis unter 1,4 Mol Anilin/pro Mol Formaldehyd, umsetzt.

Die Herstellung von sauren Kondensaten mit obigem Anilin/Formaldehyd-Molverhältnis bereitet Schwierigkeiten, da unter den notwendigen Reaktionsbedingungen (Temperatur unter 70°C) die Kondensation zur Bildung von festen Stoffen bzw. Ausscheidungen führt, die ausser negativer Beeinflussung der Qualität auch Betriebsschwierigkeiten bereiten (z.B. Verstopfung von Leitungen, Armaturen u.ä.).

Auch die Herstellung von sogenanntem Vorkondensat durch nichtkatalytische Umsetzung von Anilin mit Formaldehyd geht glatt im Bereich von bis zu 2 Mol Anilin/pro 1 Mol Formaldehyd. Wenn man Umsetzungen von unter 2 Mol Anilin/pro 1 Mol Formaldehyd durchführt, so erhält man Feststoffe sowie Schwierigkeiten beim Abscheiden von Wasser.

Es wurde nun gefunden, dass es möglich und vorteilhaft ist, einen Teil des Formaldehyds zum Zwischenkondensat zu geben. Die Zugabe kann auch während der Herstellung von Zwischenkondensat erfolgen. Das so nachbehandelte Zwischenkondensat wird dann, gegebenenfalls mehrstufig, bei höheren Temperaturen zu Methylendianilin und Methylenbrücken aufweisenden Tri- und höheren Polyarylaminen umgelagert.

Durch diese Arbeitsweise ist es möglich, Produkte mit extrem hohem Anteil am PMPPA (etwa 50%) herzustellen.

Es ist selbstverständlich möglich, auch die Zugabe von Formaldehyd in mehreren Stufen zu machen, indem man das Zwischenkondensat mehrstufig herstellt und abwechselnd Vorkondensat und Formaldehyd zu saurem Kondensat zugibt.

Das Gesamtmolverhältnis von Anilin und Formaldehyd liegt im allgemeinen in dem Bereich von 1,3:1 - 15:1, vorzugsweise 1,4:1 - 10:1 Mol Anilin pro Mol Formaldehyd.

Die Verteilung des Gesamtformaldehyds auf die Herstellung von saurem Kondensat, Vorkondensat und Zugabe zu Zwischenkondensat (Restzugabe Formaldehyd) kann auch weitgehend variiert werden:

| | | | | |
|---|---|---|---|---|
| Säurekondensat: | 0,05:0,7 | vorzugs-<br>weise | 0,2 :0,5 | Mol Formaldehyd<br>pro Mol Anilin |
| Vorkondensat: | 0,10:0,50 | -"- | 0,15:0,49 | -"- |
| Restzugabe: | 0,01:0,40 | -"- | 0,05:0,30 | -"- |

Die Restzugabe sollte bei Temperaturen von unter 70°C, vorzugsweise unter 60°C, erfolgen.

Die Reaktionszeiten und Temperaturen der Restzugabe bzw. Nachreaktion sind von der Menge des Formaldehyds und der Temperatur direkt abhängig: höhere Temperaturen sind verantwortlich für die Bildung von unerwünschten Nebenprodukten (N-Methylverbindungen).

Das Gesamtmolverhältnis von Anilin und Formaldehyd liegt im allgemeinen bei 1,5:1 bis 15:1, vorzugsweise 2:1 bis 10:1.

Die Verteilung der Ströme kann bei 1:20 bis 20:1 liegen.

Das Anilin/Formaldehyd Molverhältnis kann in den Strömen gleich wie im gesamten Prozess oder auch verschieden sein. Auch dadurch kann die Zusammensetzung der gewünschten Produkte beeinflusst werden.

Das Volumenverhältnis Anilin/Formaldehyd/Säure zu Wasser bei Herstellung von saurem Kondensat liegt im allgemeinen bei 5:1 bis 1:10. Dieser Parameter hat auch Einfluss auf die Produktqualität.

Eine Illustration der Durchführung des erfindergemässen Verfahrens zeigen nachstehende Figuren:

Die Anlage besteht aus zwei Strängen: der eine Stranz zur Umsetzung von Anilin und Formaldehyd nicht-katalytisch zu Vorkondensat und der zweite zur Umsetzung von Anilin mit Formaldehyd mit Säurekatalysator zu saurem Kondensat sowie Umsetzung des Vorkondensates zu Zwischenkondensat und anschliessende Vermischung der Ströme und/oder Endumlagerung zu MDA/PMPPA. Weiter enthält die Anlage eine Leitung für die Zugabe von Rest-Formaldehyd zum Zwischenkondensat.

Fig. 1

Die Arbeitsweise in den Strängen erfolgt in folgender Art:
Strang 1

Gemessene Ströme in Anilin und Formaldehyd werden in VR
nicht-katalytisch umgesetzt und dann in den Abscheider S geleitet, wo diese in organische und wässerige Phasen sich
trennen.

Strang 2

Ein gemessener Strom von wässeriger Anilin-Salz-Lösung, hergestellt aus Anilin und Säure im Falle von Arbeiten mit Neutralisation bzw. als Lösung erhalten aus der Aufarbeitung
der Produkte nach den Verfahren mit Austausch gegen Anilin
bzw. Extraktion mit hydrophoben Lösungsmitteln sowie Formaldehyd werden in Reaktionszone R 1 zum sauren Kondensat und
anschliessend in der Reaktionszone R 2 mit dem Vorkondensat
aus dem Abscheider S zu Zwischenkondensat umgesetzt. Anschliessend geht die Lösung in die Reaktionszone R 3 und/oder
Reaktionszone R 4 zu ein- oder mehrstufiger Umlagerung zu gewünschten Produkten.

Beim Arbeiten mit Zugabe von Restformaldehyd wird dieses in
z.B. Reaktionszone R 3 zudosiert und dann in dieser und weiteren Reaktionszonen zu gewünschten Produkten umgelagert.

Aus der Reaktionszone R 4 geht die umgelagerte Produktlösung
zur weiteren Aufarbeitung nach dem Stand der Technik.

Fig. 2

Hier wird das Vorkondensat mit einem Teil des Säurekatalysators in der Reaktionszone R 2 zu Zwischenkondensat umgesetzt
und erst dann in der Reaktionszone R 3 mit dem sauren Kondensat aus der Reaktionszone R 1 gemischt. Anschliessend wird diese Lösung in der Reaktionszone R 4 zu MDA/PMPPA umgelagert und wie üblich aufgearbeitet.

Fig. 3

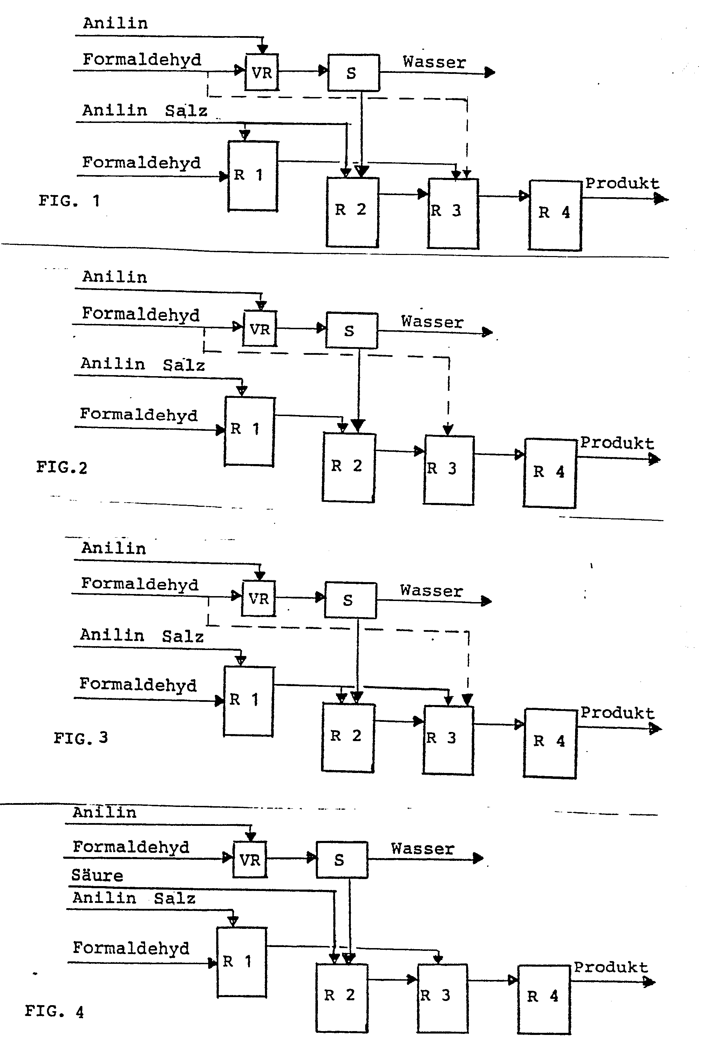

Zum Unterschied zur Arbeitsweise Fig. 2 erfolgt die Umsetzung des Vorkondensats zu Zwischenkondensat mit Teilstrom des sauren Kondensats aus R 1,und erst dann wird dieses Produkt mit dem Rest des sauren Kondensats aus R 1 in der Reaktionszone R 3 vermischt bzw. umgesetzt und gegebenenfalls erst in der Reaktionszone R 4 zu gewünschten Produkten umgelagert.

Fig. 4

Hier wird gearbeitet mit Neutralisation des Katalysators, d.h. dass freie Säure als Ausgangsprodukt benützt wird. Ein grosser Teil dieser Säure wird in Form von Anilinsalzlösungen zur Herstellung von saurem Kondensat in der Reaktionszone R 2 zu Zwischenkondensat verwendet. Die Produkte aus den Reaktionszonen R 1 und R 2 werden dann in der Reaktionszone R 3 miteinander vermischt und in dieser bzw. Reaktionszone R 4 zu MDA/PMPPA umgelagert.

Die Temperaturen in den Reaktionszonen bzw. Abscheidern werden vorzugsweise wie folgt eingestellt:

Reaktionszone V R 20 - 80°C, Abscheider S 50 - 80°C
Reaktionszone R 1 20 - 80°C
Reaktionszone R 2 30 - 100°C
Reaktionszone R 3 30 - 80°C
Reaktionszone R 4 80 - 150°C.

Die Reaktionszonen können als Rührkessel, Rührkaskade, Strömungsrohre, Kolonnen, gepackte Säulen und ähnlich ausgeführt werden.

Die Ausführung der Vorrichtungen kann selbstverständlich unabhängig von den Zeichnungen diskontinuierlich, halbkontinuierlich und ähnlich sein.

ELPROCHINE AG

<u>Beispiele 1 - 4</u>

Es werden vier Parallelversuche des erfindergemässen Verfahrens mit Salzsäure-Katalysator durchgeführt.

<u>Saure Kondensat-Herstellung</u>

Zu einer Lösung von 300 g Salzsäure 31 Gew.% (2,58 Mol) und 279 g Anilin (3 Mol) werden unter kräftiger Rührung und etwa 40°C 109,5 g 37 Gew.%iges Formaldehyd (1,35 Mol) innerhalb von etwa 20 Min. zugegeben. Anschliessend wird 30 Min. bei 50°C gehalten und dann laut Tabelle I in vier Mischgefässe verteilt. Der Ansatz entspricht einem Molverhältnis von Anilin/Formaldehyd/Salzsäure wie 1:0,45:0,88.

<u>Vorkondensat-Herstellung</u>

558 g Anilin (6 Mol) werden in einem Rührgefäss vorgelegt. Dazu gibt man bei etwa 40°C 219 g 37 Gew.% Formaldehyd (2,7 Mol) innerhalb von etwa 40 Min., ohne dass die Temperatur 50°C übersteigt. Anschliessend wird unter Rühren noch eine Stunde bei 50°C gehalten und dann abgestellt. Nach etwa 10 Min. bildet sich eine organische und wässerige Phase. Die untere Phase ist das Vorkondensat, und dieses wird laut Tabelle I zum sauren Kondensat zugegeben.

Das nach der Zugabe gebildete homogene Produkt wird anschliessend eine Stunde bei 60°C, eine Stunde bei 80°C und zwei Stunden bei 103°C gehalten.

Die Analyse erfolgt mit GLC und SE-30 als stationäre Phase auf Chromosorb und 4,4'-Methylen-bis N,N-Dimethylanilin als interner Standard zur Bestimmung des Diamingehaltes analysiert.

TABELLE I

| Versuch N | | 1 | 2 | 3 | 4 |
|---|---|---|---|---|---|
| Saures Kondensat | Mol A/F/S | 1:0,45:0,88 | 1:0,45:0,88 | 1:0,45:0,88 | 1:0,45:0,88 |
| Vorkondensat | Mol A/F | 1:0,45  – | 2:0,9  – | 3:1,35  – | 4:1,80  – |
| Summe | Mol A/F/S | 2:0,90:0,88 | 3:1,35:0,88 | 4:1,80:0,88 | 5:2,25:0,88 |
| Endprodukt | Mol A/F/S | 1:0,45:0,44 | 1:0,45:0,30 | 1:0,45:022 | 1:0,45:0,18 |
| 2,2- MDA Gew.% nicht korrigiert | | 0,05 | 0,12 | 0,24 | 0,35 |
| 2,4- MDA | | 4,1 | 6,48 | 6,74 | 7,8 |
| 4,4- MDA | | 95,5 | 93,0 | 92,6 | 91,5 |
| N-Methyl.MDA | | 0,35 | 0,40 | 0,42 | 0,35 |
| Diamin Gew.% nicht korrigiert | | 72 | 71 | 70 | 69 |
| Mol Säure/Mol FMA | | 1,0:1 | 0,66:1 | 0,49:1 | 0,40:1 |

Beispiele 5 - 8

Es werden vier Parallelversuche mit Methansulfonsäure als Katalysator durchgeführt.

Saure Kondensat-Herstellung

708,75 g 40%ige Anilin-Methansulfonatlösung in Wasser enthalten 1,5 Mol Anilin und 1,5 Mol Methansulfonsäure, werden mit 54,7 g 37 Gew.%igem Formaldehyd (0,675 Mol) bei etwa 45$^{O}$C umgesetzt und dann eine Stunde bei 50$^{O}$C gehalten. Der Ansatz entspricht einem Anilin/Formaldehyd/Methansulfonsäureverhältnis wie 1:0,45:1,0.

Anschliessend wird das saure Kondensat in vier geheizte Rührgefässe laut Tabelle II verteilt und auf 90$^{O}$C erhitzt. Nach Erreichen der Temperatur wird das Zwischenkondensat laut Tabelle zu den sauren Kondensaten zugegeben, wobei diese sich sofort homogen auflösen. Dann wird bei 90$^{O}$C eine Stunde und bei 103$^{O}$C zwei Stunden gehalten.

Vorkondensat-Herstellung

Die Herstellung des Vorkondensates erfolgt wie in Beispiel 1-4.

Zwischenkondensat-Herstellung

Das Vorkondensat wird bei etwa 40-45$^{O}$C mit 0,015 Mol 40%ige Anilin-Methansulfonatlösung in Wasser (Säurekatalysator) pro Mol Vorkondensat versetzt und eine Stunde bei etwa 50$^{O}$C gehalten. Dann wird das Zwischenkondensat, wie bereits oben erwähnt, laut Tabelle zum sauren Kondensat bei 90$^{O}$C zugegeben.

Das Produkt wird untersucht, wie das in Beispielen 1-4 beschrieben ist. Die Resultate sind aus der Tabelle II ersichtlich.

TABELLE   II

| Versuch N | | 5 | 6 | 7 | 8 |
|---|---|---|---|---|---|
| Saures Kondensat | Mol A/F/S | 3:1,35:3 | 2:0,98:2 | 1:0,45:1 | 1:0,45:1 |
| Zwischenkondensat | " A/F/S | 1:0,45:0,015 | 1:0,45:0,015 | 1:0,45:0,015 | 2:0,9 :0,03 |
| Summe | Mol A/F/S | 4:1,80:3,015 | 3:1,35:2,015 | 2:0,9 :1,015 | 3:1,35:1,03 |
| Endprodukt | Mol A/F/S | 1:0,45:0,75 | 1:0,45:0,67 | 1:0,45:0,50 | 1:0,45:0,34 |
| 2,2'- MDA | Gew.% nicht korrigiert | 0,1 | 0,1 | 0,15 | 0,30 |
| 2,4'- MDA | - " - | 2,0 | 3,75 | 4,95 | 5,95 |
| 4,4'- MDA | - " - | 97,3 | 95,6 | 94,5 | 93,5 |
| N-Methyl MDA | - " - | 0,6 | 0,55 | 0,4 | 0,25 |
| Diamin MDA | - " - | 71,0 | 68,0 | 66,0 | 63,0 |
| Mol Säure/Mol FMA | | 1,66:1 | 1,49:1 | 1,11:1 | 0,75:1 |

- 18 -

Beispiele 9 - 12

Es werden vier Parallelversuche des erfindergemässen Verfahrens mit Salzsäure-Katalysator durchgeführt.

Saure Kondensat-Herstellung

Zu einer Lösung von 309 g Salzsäure 28 Gew.% (2,41 Mol) und 256 g Anilin (2,76 Mol) werden unter kräftiger Rührung und etwa 40°C 118,0 g 31,5 Gew.%iges Formaldehyd (1,24 Mol) innerhalb von etwa 20 Min. zugegeben. Anschliessend wird 30 Min. bei 50°C gehalten und dann laut Tabelle III in vier Mischgefässe verteilt. Der Ansatz entspricht einem Molverhältnis von Anilin/Formaldehyd/Salzsäure wie 1:0,45:0,88.

Vorkondensat-Herstellung

409,2 g Anilin (4,4 Mol) werden in einem Rührgefäss vorgelegt. Dazu gibt man bei etwa 40°C 189 g 31,5 Gew.%iges Formaldehyd (1,98 Mol) innerhalb von etwa 40 Min., ohne dass die Temperatur 50°C übersteigt. Anschliessend wir unter Rühren noch 30 Min. bei etwa 40°C gehalten und dann auf 60°C erhitzt werden und nach etwa 10 Min. abgestellt. Es bilden sich eine organische und eine wässerige Phase. Die untere Phase ist das Vorkondensat, und dieses wird laut Tabelle III zum sauren Kondensat zugegeben und etwa 15 Min. bei 50°C gehalten.
Anschliessend wird laut Tabelle III Restformaldehyd bei 50°C während etwa 10 Min. zugegeben.
Das nach der Zugabe gebildete Produkt wird dann eine Stunde bei 60°C, eine Stunde bei 80°C und zwei Stunden bei 103°C gehalten.

Die Analyse erfolgt mit GLC und SE-30 als stationäre Phase auf Chromosorb und 4,4'-Methylen- bis N,N-Dimethylanilin als interner Standard zur Bestimmung des Diamingehaltes.

TABELLE III

| Versuch Nr. | | 9 | 10 | 11 | 12 |
|---|---|---|---|---|---|
| Saures Kondensat Mol A/F/S | | 1:0,45:0,88 | 1:0,45:0,88 | 1:0,45:0,88 | 1:0,45:0,88 |
| Vorkondensat | Mol A/F | 1:0,45 – | 1:0,45 – | 1:0,45 – | 1:0,45 – |
| Summe | Mol A/F/S | 2:0,90:0,88 | 1:0,90:0,88 | 1:0,90:0,88 | 1:0,90:0,88 |
| Zwischenkondensat Mol A/F/S | | 1:0,45:0,44 | 1:0,45:0,44 | 1:0,45:0,44 | 1:0,45:0,44 |
| Restzugabe FMA | Mol | – | 0,05 | 0,10 | 0,15 |
| Endprodukt | Mol A/F/S | 1:0,45:0,44 | 1:0,50:0,44 | 1:0,55:0,44 | 1:0,60:0,44 |
| 2,2-MDA Gew.% | | – | – | – | – |
| 2,4-MDA | | 3,7 | 3,1 | 2,4 | 2,2 |
| 4,4-MDA | | 96,3 | 96,9 | 97,6 | 98,1 |
| N-Methyl.MDA | | 0,5 | 0,5 | 0,5 | 0,5 |
| Diamin Gew.% | | 69,0 | 65,2 | 57,8 | 50,0 |
| Polyamin Gew.% (3-Kern und höher) | | 31,0 | 34,8 | 42,2 | 50,0 |

<u>Beispiele 13 - 16</u>

Es werden vier Parallelversuche des erfindergemässen Verfahrens mit Salzsäure-Katalysator durchgeführt.

<u>Saure Kondensat-Herstellung</u>

Zu einer Lösung von 103 g Salzsäure 31,5 Gew.% (0,9 Mol) und 93 g Anilin (1 Mol) werden unter kräftiger Rührung und etwa 40°C 43 g 31,5 Gew.%iges Formaldehyd (0,45 Mol) innerhalb von etwa 20 Min. zugegeben. Anschliessend wird 30 Min. bei 50°C gehalten und dann laut Tabelle II in vier Mischgefässe verteilt. Der Ansatz entspricht einem Molverhältnis von Anilin/Formaldehyd/Salzsäure wie 1:0,45:0,9).

<u>Vorkondensat-Herstellung</u>

409,2 g Anilin (4,4 Mol) werden in einem Rührgefäss vorgelegt. Dazu gibt man bei etwa 40°C 189 g 31,5 Gew.%iges Formaldehyd (1,98 Mol) innerhalb von etwa 40 Min., ohne dass die Temperatur 50°C übersteigt. Anschliessend wird unter Rühren noch 30 Min. bei 40°C gehalten und dann auf 60°C erhitzt und nach etwa 10 Min. abgestellt. Es bilden sich eine organische und eine wässerige Phase. Die untere Phase ist das Vorkondensat, und dieses wird laut Tabelle IV zum sauren Kondensat zugegeben und etwa 15 Min. bei 50°C gehalten.
Anschliessend wird laut Tabelle II Restformaldehyd bei 50°C während etwa 10 Min. zugegeben.
Das nach der Zugabe gebildete Produkt wird dann eine Stunde bei 60°C, eine Stunde bei 80°C und zwei Stunden bei 103°C gehalten.

Die Analyse erfolgt mit GLC und SE-30 als stationäre Phase auf Chromosorb und 4,4'-Methylen- bis N,N-Dimethylanilin als interner Standard zur Bestimmung des Diamingehaltes.

TABELLE IV

| Versuch Nr. | | 13 | 14 | 15 | 16 |
|---|---|---|---|---|---|
| Saures Kondensat | Mol A/F/S | 1:0,45:0,90 | 1:0,45:0,90 | 1:0,45:0,90 | 1:0,45:0,90 |
| Vorkondensat | Mol A/F | 5:2,25  - | 5:2,25  - | 5:2,25  - | 5:2,25  - |
| Summe | Mol A/F/S | 6:2,70:0,90 | 6:2,70:0,90 | 6:2,70:0,90 | 6:2,70:0,90 |
| Zwischenkondensat | Mol A/F/S | 1:0,45:0,15 | 1:0,45:0,15 | 1:0,45:0,15 | 1:0,45:0,15 |
| Restzugabe FMA | Mol | - | 0,05 | 0,10 | 0,15 |
| Endprodukt | Mol A/F/S | 1:0,45:0,15 | 1:0,50:0,15 | 1:0,55:0,15 | 1:0,60:0,15 |
| 2,2-MDA Gew.% nicht korrigiert | | 0,5 | 0,5 | 0,5 | 0,5 |
| 2,4-MDA | | 8,6 | 6,1 | 7,3 | 7,1 |
| 4,4-MDA | | 91,4 | 91,9 | 92,7 | 92,8 |
| N-Methyl.MDA | | 0,5 | 0,5 | 0,5 | 0,5 |
| Diamin   Gew.% nicht korrigiert | | 68,3 | 62,7 | 56,1 | 52,0 |
| Polyamin Gew.% (3-Kern und höher) | | 31,7 | 37,3 | 43,9 | 48 |

0109931

PATENTANSPRÜCHE

1. Verfahren zur Herstellung von Gemischen aus Methylendianilin sowie Polymethylen-Polyphenylen-Polyaminen durch Kondensation von Anilin und Formaldehyd in Gegenwart eines Säurekatalysators mit steuerbarem Gehalt an 2,4'-Methylendianilin im Zweikern und steuerbarem Gehalt an Methylendianilin im Gesamtgemisch und/oder reduzierter Säurekatalysator-Menge, dadurch gekennzeichnet, daß man die Umsetzung von mindestens zwei Strömen durchführt:

a. Einen Teil Formaldehyd und Anilin in Anwesenheit von gegebenenfalls gesamtem Säurekatalysator zu saurem Kondensat, im wesentlichen bestehend aus Aminobenzylamin und/oder Methylendianilin und oligomeren Polymethylen-Polyphenylen-Polyaminen umsetzt,

b. das restliche Anilin und Teil oder das gesamte restliche Formaldehyd entweder nicht-katalytisch zu sogenanntem Vorkondensat umsetzt, gegebenenfalls das Formaldehyd und Reaktionswasser abtrennt und gegebenenfalls mit dem Rest des Säurekatalysators, einen Teil oder das gesamte saure Kondensat zum Zwischenkondensat umlagert oder katalytisch direkt zum Zwischenkondensat in Anwesenheit von schwachen Säuren, Kohlensäure, sauren Ionenaustauschern, festen Katalysatoren, Salzen, starken Säuren mit kurzen Verweilzeiten, thermisch bei Temperaturen von über 100°C u.ä. umsetzt und

c. das saure Kondensat und Vorkondensat oder das Zwischenkondensat, gegebenenfalls zusammenmischt und gegebenenfalls nach Zugabe von Restformaldehyd gegebenenfalls mehrstufig zu Methylendianilin, sowie oligomerem Polymethylen, Polyphenylen-Polyaminen umlagert und nach dem Stand der Technik aufarbeitet.

2. Verfahren nach Anspruch 1, dadurch gekennzeichnet, daß man mit verschiedenen Molverhältnissen Anilin zu Formaldehyd in den Strömen arbeitet,

3. Verfahren nach einem der Ansprüche 1 und 2, dadurch gekennzeichnet, daß das Molverhältnis zwischen saurem Kondensat und Vorkondensat von 0,001:25 bis 10:0,1 beträgt,

4. Verfahren nach einem der Ansprüche 1 und 2, dadurch gekennzeichnet, daß das Molverhältnis zwischen saurem Kondensat und Zwischenkondensat 0,001:25 bis 10:0,1 beträgt,

5. Verfahren nach einem der Ansprüche 1, 2 und 4, dadurch gekennzeichnet, daß Vorkondensat zu Zwischenkondensat mit 0,001 - 0,3 Mol Säurekatalysator bzw. Salzlösung umgelagert wird,

6. Verfahren nach einem der Ansprüche 1 - 4, dadurch gekennzeichnet, daß die Umlagerung des Vorkondensats zum Zwischenkondensat mit 0,001 - 0,5 vorzugsweise 0,01 - 0,1 Mol saurem Kondensat erfolgt,

7. Verfahren nach einem der Ansprüche 1 - 6, dadurch gekennzeichnet, daß man die Restformaldehydmenge-Zugabe in mehreren Teilen abwechselnd mit Vorkondensat zugibt,

8. Verfahren nach einem der Ansprüche 1 - 7, dadurch gekennzeichnet, daß man die Restformaldehydzugabe bei Temperaturen von unter 60°C durchführt,

9. Verfahren nach einem der Ansprüche 1 - 8, dadurch gekennzeichnet, daß man die Umsetzung insgesamt bis in vier Stufen durchführt, wobei die Reaktionstemperatur in der ersten Stufe 20 bis 60°C, in der zweiten Stufe 50 - 70°C, in der dritten Stufe 60 - 90°C und in der vierten Stufe 90 - 125°C und die Verweilzeit in jeder Stufe von 0,1 bis 2 Stunden beträgt.

0109931

Anilin

Formaldehyd → VR → S → Wasser

Anilin Salz

Formaldehyd → R 1 → R 2 → R 3 → R 4 → Produkt

FIG. 1

Anilin

Formaldehyd → VR → S → Wasser

Anilin Salz

Formaldehyd → R 1 → R 2 → R 3 → R 4 → Produkt

FIG.2

Anilin

Formaldehyd → VR → S → Wasser

Anilin Salz

Formaldehyd → R 1 → R 2 → R 3 → R 4 → Produkt

FIG. 3

Anilin

Formaldehyd → VR → S → Wasser

Säure

Anilin Salz

Formaldehyd → R 1 → R 2 → R 3 → R 4 → Produkt

FIG. 4